# EUROPEAN PATENT APPLICATION

(11) **EP 4 682 266 A1**
(43) Date of publication of application: **21.01.2026**
(21) Application number: 24774009.5
(22) Date of filing: 15.03.2024
(51) Int. Cl.: C12Q 1/68, C12N 5/10, C12N 1/15

(54) **CELL HAVING REPORTER GENE SYSTEM OF DUAL-STRUCTURE CHROMOGENIC ENZYME AND USE THEREOF**

(30) Priority: 17.03.2023 US 202363490777 P
(71) Applicant: Kaohsiung Medical University, Kaohsiung 807 (TW)
(72) Inventor: LIN, Wen-Wei, Kaohsiung City Taiwan 807 (CN); CHENG, Tian-Lu, Kaohsiung City Taiwan 807 (CN); LIU, En-Shuo, Kaohsiung City Taiwan 807 (CN); CHANG, Mu-Shen, Kaohsiung City Taiwan 807 (CN); LEE, Chia-Yi, Kaohsiung City Taiwan 807 (CN)
(74) Representative: Lang, Christian
(86) International application number: PCT/CN2024/081816
(87) International publication number: WO 2024/193443

(57) **Abstract**

Provided is a cell having a reporter gene system of a dual structure chromogenic enzyme, wherein the cell has the function of expressing a first chromogenic enzyme on the cell surface and releasing a second chromogenic enzyme. Further provided is a method for screening a candidate to be tested by using the cell, containing contacting the candidate to be tested with the cell, and measuring a reaction signal of the chromogenic enzymes generated by the cell to analyze the characteristics of the candidate to tested.

## Description

### FIELD OF THE INVENTION

The present invention relates to a cell having a reporter gene system of a dual-structure chromogenic enzyme and a screening method thereof, wherein the cell can express a first chromogenic enzyme on the cell surface and release a second chromogenic enzyme, and the cell can be used for high-throughput screening of drugs or compounds.

### BACKGROUND OF THE INVENTION

The highly sensitive "dual luciferase reporter assay" is the most widely used and widely accepted reporter gene among currently available reporter genes, and the assay includes luciferases from two different species, the Eastern firefly/*Photinus pyralis*) and the Renilla/*Renilla reniformis.* However, there are some issues about the assay need to be improved: (1) The substrates used in the assay are firefly luciferin and coelenterazine, and chemical syntheses of these two substrates require precious metals, making the assay an expensive one. The cost becomes a major issue when large quantities of experiments are repeatedly conducted for high-throughput screening; (2) Luciferase is expressed in the cell, the substrate must first pass through or disrupt the cell membrane before the enzyme and substrate come into contact with each other for catalyzation; and (3) Luciferase has a short half-life, and the chemical properties of luciferin/coelenterazine are not stable enough. If a laboratory needs to screen drugs or compounds on a long-term basis, the instability of the enzyme and substrates increases the analytical variability of subsequent experimental results.

### DESCRIPTION OF THE INVENTION

Chromogenic enzymes can catalyze the oxidation of specific compounds (substrates), breaking the bond between functional groups to change the optical activity. The color change characteristics allow direct observation of the color change before and after. As a result, they are commonly used in research or diagnosis, for example, in immunoassays and detection techniques such as enzyme-linked immunosorbent assay (ELISA) or Western blot. The present invention can express chromogenic enzymes on cell membranes to form membrane-tethered chromogenic enzymes (MTCE), and can also directly secrete chromogenic enzymes into the supernatant of a cell growth environment to form secreted chromogenic enzymes (SCE), allowing the substrates (for example, 2,2'-azino-bis(3-ethylbenzothiazoline-6-sulfonic acid (ABTS), 3,3',5,5'-tetramethyl[1,1'-biphenyl]-4,4'-diamine (TMB), and Luminol) to react and develop colors without penetrating the cell membranes or disrupting the cells. Therefore, the cells can be directly labeled and transfected cells can be identified without relying on structural proteins or organelles in the cells.

The present invention designs a reporter gene system of a dual-structure chromogenic enzyme (DSCE). First, the order of the sequence of the MTCE reporter gene of the present invention is designed as follows: inducible promoter (IP) - signal peptide (SP) - chromogenic enzyme (CE) - tag (Tag) - transmembrane domain (TM). The order of the sequence of the SCE reporter gene is designed as follows: continuously expressed promoter (CEP) - signal peptide - chromogenic enzyme - tag. In the present invention, the inducible promoter can be an antioxidant response element (ARE); the chromogenic enzyme can be horseradish peroxidase (HRP); the tag can be myc; the transmembrane domain can be mB7; and the continuously expressed promoter can be CMV.

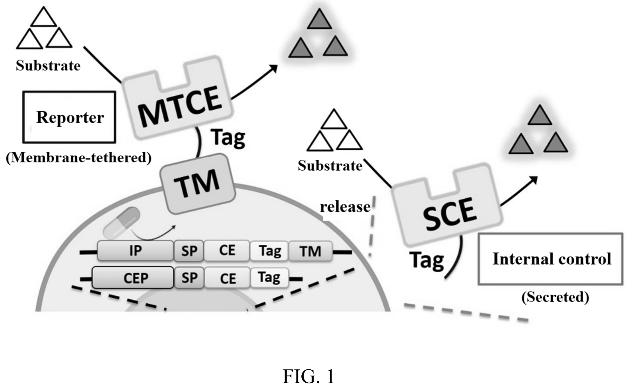

As shown in FIG 1, a plasmid having a MTCE reporter gene and a plasmid having a SCE reporter gene are co-transfected into a cell. Therefore, the transfected cell has two kinds of reporter genes. When a candidate to be tested (for example, a drug or a compound) is applied to the cell, the promoter can be initiated so that the subsequent chromogenic enzyme is produced through transcription and translation. Therefore, researchers can determine whether the candidate to be tested is a promoting or inhibitory molecule by observing upregulation or downregulation of the chromogenic enzyme's characteristics (compared to a negative control).

Regarding the design structure of MTCE, a tag is placed between a chromogenic enzyme and a transmembrane domain. Furthermore, the signal peptide and transmembrane domain on the MTCE assist in expressing the chromogenic enzyme on the cell membrane, forming a membrane-expressed chromogenic enzyme. The MTCE acts as a "reporter" in the DSCE reporter gene system.

In the present invention, the SCE reporter gene includes a CEP, which is continuously activated in the cell, causing the subsequent chromogenic enzyme to be produced through transcription and translation. Regarding the design structure of the SCE, there is a tag behind the chromogenic enzyme. In addition, the signal peptide on the SCE assists in releasing the chromogenic enzyme from the cell, forming a secreted chromogenic enzyme. The SCE serves as an "internal control" in the DSCE reporter gene system. Therefore, before conducting a chromogenic assay for screening a drug or a compound, the supernatant containing the SCE is removed first. Because the supernatant includes SCE and "culture medium" used to culture cells, after a substrate is added, the color of the substrate changes. An ELISA analyzer is used to read the values, and detect the substrate's specific maximum absorbance wavelength to obtain the signal and determine whether the transfection efficiencies of all experimental groups are the same. Then, the substrate is added to the cell that expresses MTCE, causing the color of the substrate to change. An ELISA analyzer is used to read the values and detect the specific maximum absorption wavelength of the substrate to obtain signals for determining whether the drug or compound is promoting or inhibitory.

In addition, the stability of the chromogenic enzyme and its substrate is high, and after catalyzing the substrate, the chromogenic enzyme also has high stability. Therefore, when carrying out a chromogenic assay, after adding a substrate into a plurality of 96-well plates, an ELISA analyzer can be used to carry out a long-term and continuous value reading to obtain accurate data. The present invention believes that this technology is applicable to research and development units that require a large number of experiments to be undertaken, making it a low-cost, highly sensitive reporter gene system to improve the efficiency of drug development and provide revolutionary breakthrough of the reporter gene system.

The present invention provides a polynucleotide which comprises a first gene set and a second gene set, wherein the first gene set comprises a first promoter, a nucleic acid sequence encoding a first signal peptide, a nucleic acid sequence encoding a first chromogenic enzyme, and a nucleic acid sequence encoding a transmembrane domain; and the second gene set comprises a second promoter, a nucleic acid sequence encoding a second signal peptide, and a nucleic acid sequence encoding a second chromogenic enzyme.

The term "polynucleotide" is used interchangeably herein and refers to a polymer of nucleotides of any length, and comprises DNA and RNA.

In one embodiment, the first promoter and the second promoter are inducible promoters or continuously expressed promoters. In another embodiment, the first promoter is an inducible promoter. In another embodiment, the second promoter is a continuously expressed promoter.

In another embodiment, the inducible promoter comprises an antioxidant response element (ARE), TEAD1, NF-κB, Myc, Klf-4, Sox2, Oct4, a hypoxia response element, FOXO3a, Sip-1, or Slug. In one embodiment, the continuously expressed promoter comprises EFS, CMV, CK8, MHC, MYOD, hTERT, SRα, SV40, LTR, CAG, or RSV.

In one embodiment, the first chromogenic enzyme and the second chromogenic enzyme comprise horseradish peroxidase, alkaline phosphatase (AP), glucose oxidase, β-lactamase or β-galactosidase.

In another embodiment, the transmembrane domain comprises mB7, CD63, CD81, CD9, CD82, CD44, CD47, CD55, LAMP2B, ICAM, integrin, ARRDC1 or annexin.

In another embodiment, the first gene set and the second gene set further comprise a tag, respectively. In a preferred embodiment, the tag comprises a Myc tag, a HAT tag, a HA tag, a TAP tag, a GST tag, a chitin binding domain (CBD tag), a maltose binding protein (MBP tag), a Flag tag, a Strep tag or a His tag.

As used herein, the term "signal peptide" refers to an amino acid sequence that can guide a downstream polypeptide chain into the endoplasmic reticulum membrane. In some aspects, the components on the first gene set are arranged in the order of: the first promoter, the nucleic acid sequence encoding the first signal peptide, the nucleic acid sequence encoding the first chromogenic enzyme, the tag, and the nucleic acid sequence encoding the transmembrane domain. In addition, the components on the second gene set are arranged in the order of: the second promoter, the nucleic acid sequence encoding the second signal peptide, the nucleic acid sequence encoding the second chromogenic enzyme, and the tag. Therefore, the polypeptide sequences of the first chromogenic enzyme, the transmembrane domain, the second chromogenic enzyme and the tag are all located downstream of the signal peptide, so that these polypeptide sequences can be guided into the endoplasmic reticulum.

The present invention further provides a vector, which comprises the polynucleotide.

As used herein, the term "vector" refers to a construct that is capable of delivering and usually expressing one or more genes or sequences of interest in a host cell. Examples of vectors include, but are not limited to, a viral vector, naked DNA or RNA expression vector, plasmid, cosmid or phage vector, DNA or RNA expression vector associated with a cationic condensing agent, and DNA or RNA expression vector encapsulated in a liposome.

The present invention further provides a method for producing a host cell having a reporter gene system of a dual-structure chromogenic enzyme, which comprises (a) constructing a vector having a polynucleotide, wherein the polynucleotide comprises a first gene set and a second gene set, wherein the first gene set comprises a first promoter, a nucleic acid sequence encoding a first signal peptide, a nucleic acid sequence encoding a first chromogenic enzyme, and a nucleic acid sequence encoding a transmembrane domain; and the second gene set comprises a second promoter, a nucleic acid sequence encoding a second signal peptide, and a nucleic acid sequence encoding a second chromogenic enzyme; (b) transfecting the vector of step (a) into a host cell; and (c) screening the host cell that expresses the first chromogenic enzyme on the cell membrane and releases the second chromogenic enzyme.

As used herein, the term "transfection" refers to introduction of exogenous nucleic acid into an eukaryotic cell. Transfection can be achieved by various means known in the art, including calcium phosphate-DNA coprecipitation, DEAE-dextran-mediated transfection, polybrene-mediated transfection, electroporation, microinjection, liposome fusion, liposome transfection, plasmid fusion, retroviral infection, and gene gun technology (gene gun).

Furthermore, the present invention provides a host cell, which comprises the polynucleotide or comprises a vector containing the polynucleotide.

The present invention provides a cell having a reporter gene system of a dual-structure chromogenic enzyme, which comprises a vector containing a polynucleotide, the polynucleotide comprises a first gene set and a second gene set, wherein the first gene set comprises a first promoter, a nucleic acid sequence encoding a first signal peptide, a nucleic acid sequence encoding a first chromogenic enzyme, and a nucleic acid sequence encoding a transmembrane domain; and the second gene set comprises a second promoter, a nucleic acid sequence encoding a second signal peptide, and a nucleic acid sequence encoding a second chromogenic enzyme. In addition, the present invention also provides a cell having a reporter gene system of a dual-structure chromogenic enzyme, wherein the cell surface of the cell expresses the first chromogenic enzyme, and the cell has a function of releasing the second chromogenic enzyme.

In addition, the present invention further provides a method for screening a candidate to be tested by using a cell having a reporter gene system of a dual-structure chromogenic enzyme, which comprises: (1) providing a cell having a reporter gene system of a dual-structure chromogenic enzyme, and placing the cell in a culture medium, wherein the cell comprises a vector comprising a polynucleotide, the polynucleotide comprises a first gene set and a second gene set, wherein the first gene set comprises a first promoter, a nucleic acid sequence encoding a first signal peptide, a nucleic acid sequence encoding a first chromogenic enzyme, and a nucleic acid sequence encoding a transmembrane domain; and the second gene set comprises a second promoter, a nucleic acid sequence encoding a second signal peptide, and a nucleic acid sequence encoding a second chromogenic enzyme; (2) contacting at least one candidate to be tested with the cell, wherein the at least one candidate to be tested promotes expression of the first promoter and the second promoter in the polynucleotide, and encodes the following proteins: the first signal peptide, the first chromogenic enzyme, the transmembrane domain, the second signal peptide, and the second chromogenic enzyme, wherein the first signal peptide and the transmembrane domain cause the first chromogenic enzyme to be expressed on the cell membrane of the cell, and the second signal peptide causes the second chromogenic enzyme to be released from the cell into the culture medium; (3) centrifuging the culture medium to separate a separated solution comprising the cell having the first chromogenic enzyme expressed on the cell membrane and a supernatant comprising the second chromogenic enzyme; (4) adding a first substrate to the separated solution to perform a first chromogenic reaction with the first chromogenic enzyme, and adding a second substrate to the supernatant to perform a second chromogenic reaction with the second chromogenic enzyme; and detecting signals of the first chromogenic reaction and the second chromogenic reaction, and selecting the signals of at least one chromogenic reaction to analyze characteristics of the at least one candidate to be tested.

In some aspects, the cell is a host cell. In the instant specification, the type of the term "host cell" and "cell" can use an eukaryotic cell such as a mammalian cell, an insect cell, a plant cell, a fungal cell, etc., or a prokaryotic cell such as an *Escherichia coli* (*E. Coli*), a *Bacillus subtilis,* etc. In one embodiment, the host cell and the cell comprise a mammalian cell. In a preferred embodiment, the mammalian cell comprises a CHO cell, HEK293T cell, HEK293 cell, HeLa cell, BHK21 cell or Vero cell.

In one embodiment, the culture medium comprises a cell culture medium.

In the present invention, the candidate to be tested can be any substances. Examples of the candidate to be tested comprises a drug, compound, extract of a natural product, nucleic acid molecule, and protein. In another embodiment, the at least one candidate to be tested comprises a compound or a protein.

In the present invention, the functional design of the first gene set is to allow the first chromogenic enzyme to be expressed on the cell membrane of the host cell. Therefore, the first gene set comprises a nucleic acid sequence encoding a transmembrane domain. The first chromogenic enzyme is carried to the cell membrane of the cell for expression through the first signal peptide and the transmembrane domain. The functional design of the second gene set is to release the second chromogenic enzyme from the host cell into the culture medium. After the cell membranes of the cell expresses the first chromogenic enzyme and the host cell releases the second chromogenic enzyme, the culture medium is centrifuged to separate two solutions: a separated solution containing the cell and a supernatant containing the second chromogenic enzyme (excluding the cell).

In the present invention, a substrate is added to the separated solution and the supernatant, respectively, to perform chromogenic reactions with the first chromogenic enzyme and the second chromogenic enzyme, and signals of the chromogenic reactions can be detected and quantified. In one embodiment, the first substrate and the second substrate comprise ABTS, TMB, 3,3'-diaminobenzidine tetrahydrochloride (DAB), 4-chloro-1-naphthol (4-CN), 3-amino-9-ethyl carbazole (AEC), enhanced chemiluminescence solution (ECL solution), or luminol.

In some aspects, the signals of the chromogenic reactions are changes in the expression level of the product produced after the chromogenic enzyme reacts with the substrate. In the present invention, the method for detecting the chromogenic reactions is not limited. The present invention can use any known immunoassays to detect the signals of chromogenic reaction. In another embodiment, the detection method of the step (5) comprises an immunoassay, wherein the immunoassay comprises direct ELISA, indirect ELISA, sandwich ELISA, western blot analysis, competitive ELISA, immunohistochemical staining, lateral flow assay, multiplex immunoassay, radioimmunoassay, immunoradiometric assay or fluorescence spectrometer. The present invention achieves the effect of rapid analysis and quantification of the candidate to be tested by detecting signals generated after the chromogenic enzyme reacts with the substrate.

The cell having a reporter gene system of a dual-structure colorimetric enzyme of the present invention can be used to analyze one candidate to be tested or two different candidates to be tested. When only one candidate to be tested is used to treat the cell, the present invention detects two sets of chromogenic reaction signals, wherein one set of signal changes is used to analyze cell transfection efficiency of the vector, and the other set of signal changes is used to analyze characteristics of the candidate to be tested. For example, the chromogenic reaction signals of the second chromogenic enzyme can be selected as a transfection efficiency control group for determining the cell transfection efficiency; and the chromogenic reaction signals of the first chromogenic enzyme are used to analyze the characteristics of the candidate to be tested. In one embodiment, when the at least one candidate to be tested is one single candidate to be tested, the step (5) comprises selecting signals of one chromogenic reaction for analyzing cell transfection efficiency of the vector; and signals of another chromogenic reaction for analyzing the characteristics of the at least one candidate to be tested. Furthermore, the present invention can treat the cell with two different candidates to be tested, for example, a first candidate to be tested and a second candidate to be tested. When performing signal analysis of the chromogenic reactions, one set of signal changes is used to analyze the characteristics of the first candidate to be tested, and the other set of signal changes is used to analyze the characteristics of the second candidate to be tested. In another embodiment, when the at least one candidate to be tested comprises a first candidate to be tested and a second candidate to be tested, the step (5) comprises selecting signals of one chromogenic reaction for analyzing the characteristics of the first candidate to be tested, and signals of the other chromogenic reaction for analyzing the characteristics of the second candidate to be tested.

The present invention can determine whether the candidate to be tested is a promoting molecule or an inhibitory molecule based on changes in the signals of the chromogenic reaction. When the signals of the chromogenic reaction of the candidate to be tested are higher than signals of a chromogenic reaction of a control group, the candidate to be tested is a promoting molecule; otherwise, when the signals of the chromogenic reaction of the candidate to be tested are lower than the signals of the chromogenic reaction of the control group, the candidate to be tested is an inhibitory molecule. In one embodiment, the step (5) further comprises determining whether the at least one candidate to be tested is a promoting molecule or an inhibitory molecule based on a characteristic analysis of the at least one candidate to be tested, wherein the determining method is that when the signals of the chromogenic reaction of the at least one candidate to be tested are higher than signals of a chromogenic reaction of a control group, the at least one candidate to be tested is a promoting molecule; otherwise, when the signals of the chromogenic reaction of the at least one candidate to be tested are lower than the signals of the chromogenic reaction of the control group, the at least one candidate to be tested is an inhibitory molecule.

As used herein, a control group refers to a group to which a drug, candidate to be tested or known inducer/inhibitor is added or not added in an ongoing experiment. For example, when the present invention is to screen for an inhibitory compound, an inducer is added to the control group, and vice versa. If the candidate to be tested itself continues to express, then screening for an inhibitory compound can be performed directly without additional addition of an inducer.

Therefore, the DSCE reporter gene system of the present invention has five major advantages: (1) high sensitivity; (2) low cost of a single test; (3) a long-lasting signal after the chromogenic enzyme catalyzes the substrate, the signal is stable for about 2 hours and can continuously detect a plurality of 96-well plates; (4) the substrate can react with the chromogenic enzyme on the cell membrane or in the supernatant without lysing the cells, which can simplify the operation steps; and (5) the substrate can contact the chromogenic enzyme directly without penetrating the cell membrane, which can achieve high reaction efficiency.

### BRIEF DESCRIPTION OF THE DRAWINGS

FIG. 1 illustrates a reporter gene system of a dual-structure chromogenic enzyme according to the present invention. MTCE: membrane-tethered chromogenic enzyme; SCE: secreted chromogenic enzyme; IP: inducible promoter; SP: signal peptide; CE: chromogenic enzyme; TM: transmembrane domain; and CEP: continuously expressed promoter.

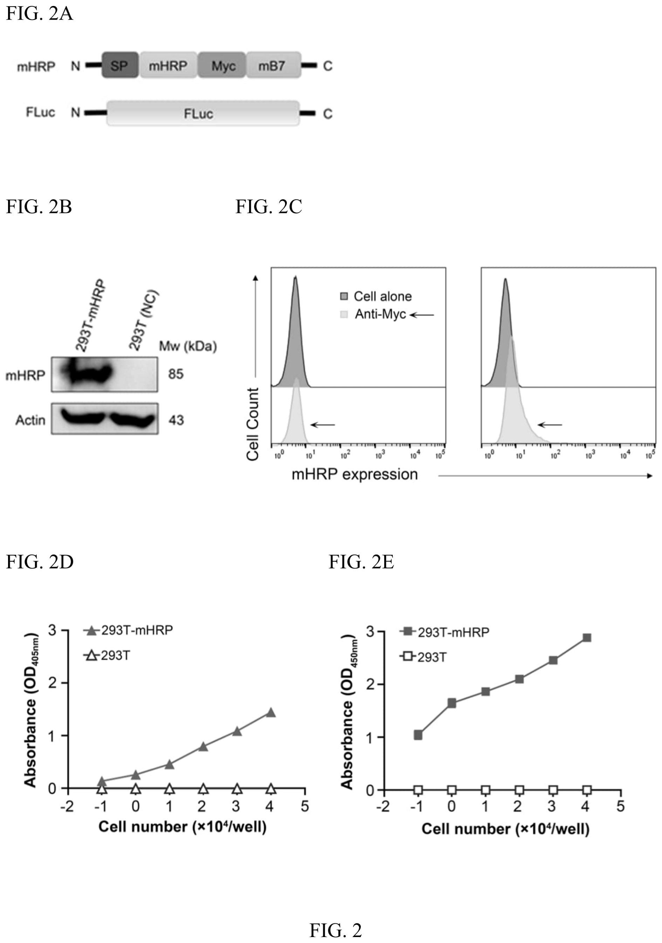

FIG. 2 shows experimental results showing that membrane-tethered horseradish peroxidase (mHRP) has the correct molecular weight of 85 kDa, is membrane expressed, and has enzyme activity. FIG. 2A is a schematic diagram of the mHRP protein sequence. The mHRP sequence is inserted into a pLNCX-CMV vector: starting from the N-terminus, the Igκ signal peptide (SP), mHRP, Myc (tag extracellular domain), and mB7 (transmembrane and intracellular domain). FIG. 2B shows that 293T-mHRP is produced for transient expression of mHRP. Protein expression of mHRP is analyzed by western blot. Lane 1: 293T-mHRP; Lane 2: 293T cells as a negative control (NC). Actin is used as a control group for total number of cells. FIG. 2C shows membrane expression of mHRP detected by Myc tag and corresponding secondary antibodies, and analysis is carried out by a flow cytometer. 293T-mHRP or 293T cells alone is used as a control group for unstained antibodies (dark gray); anti-Myc antibodies and corresponding secondary antibodies are stained (light gray, arrows indicate the groups). FIGs. 2D and 2E show functional analysis of 293T-mHRP, respectively. 1.6 × 10⁵ 293T-mHRP cells are subjected to serial dilution (two-fold dilution). 293T cells (hollow triangles or hollow squares) and 293T-mHRP (solid triangles or solid squares) are detected for ABTS (FIG. 2D) or TMB (FIG. 2E) at OD₄₀₅ₙₘ or OD₄₅₀ₙₘ, respectively. All x-axes are converted to a log₂ scale. Number is 3, and three independent replicate experiments are conducted. FLuc: Firefly luciferase.

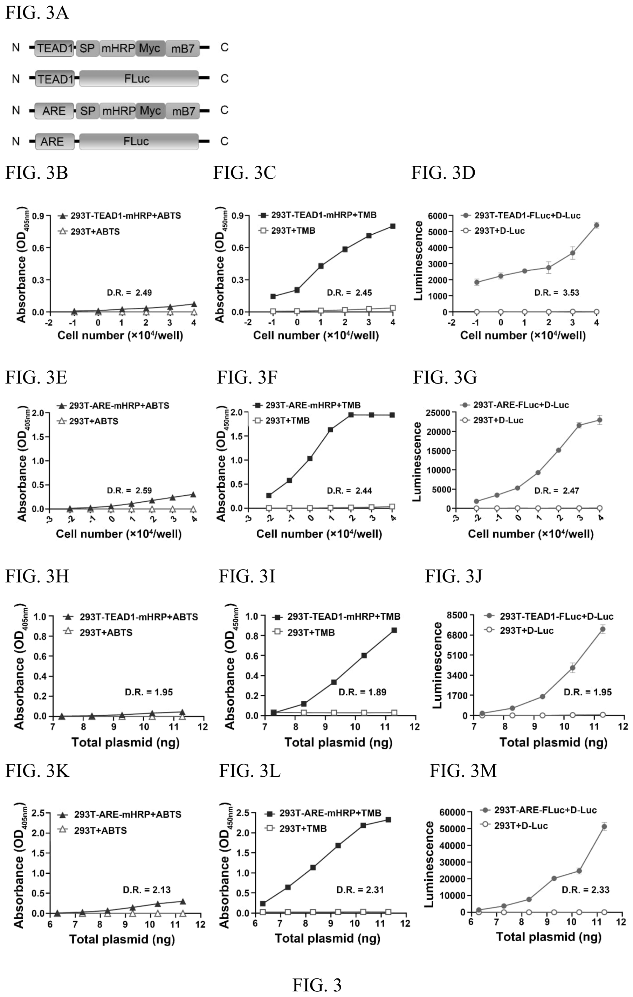

FIG. 3 shows a comparison of the sensitivity of the mHRP reporter gene and the FLuc reporter gene by diluting the number of cells or the total amount of plasmids. FIG. 3A shows a schematic diagram of the protein sequences of pTEAD1-mHRP, pTEAD1-FLuc, pARE-mHRP, and pARE-FLuc. The mHRP sequence (from SP to mB7) from pLNCX-CMV-mHRP is inserted into the pTEAD1 and pARE vectors. FIGs. 3B to 3G show two-fold dilution (6 or 7 dilution points) of 1.6 × 10⁵ pTEAD1 (or pARE) cells containing mHRP or FLuc. For functional analysis of mHRP and FLuc, ABTS or TMB are detected at OD₄₀₅ₙₘ or OD₄₅₀ₙₘ, respectively, and D-luciferin (D-Luc) is detected at a preset fluorescence wavelength of 562 nm. Optical signals are detected by using an ELISA analyzer or fluorimeter. The signals produced by the reaction of mHRP cells and TMB is higher than that produced by ABTS, indicating that TMB is more sensitive than ABTS. In the "diluted total number of cells sensitivity test assay," sensitivity is defined as the "minimum" total number of cells required to produce a "discernible" signal. FIGs. 3B to 3D show the signal detection sensitivities of pTEAD1-mHRP-ABTS, pTEAD1-mHRP-TMB, and pTEAD1-FLuc-D-Luc, respectively. A comparison of pTEAD1-mHRP-ABTS (293T-TEAD1-mHRP + ABTS) (FIG. 3B), pTEAD1-mHRP-TMB (293T-TEAD1-mHRP + TMB) (FIG. 3C), and pTEAD1-FLuc-D-Luc (293T-TEAD1-FLuc + D-Luc) (FIG. 3D) reveal that pTEAD1-mHRP-TMB and pTEAD1-FLuc-D-Luc have similar signal detection sensitivity (5×10³ cells). FIGs. 3E to 3G show the signal detection sensitivities of pARE-mHRP-ABTS, pARE-mHRP-TMB, and pARE-FLuc-D-Luc, respectively. A comparison of pARE-mHRP-ABTS (293T-ARE-mHRP + ABTS) (FIG. 3E), pARE-mHRP-TMB (293T-ARE-mHRP + TMB) (FIG. 3F), and pARE-FLuc-D-Luc (293T-ARE-FLuc + D-Luc) (FIG. 3G) reveal that pARE-mHRP-TMB and pARE-FLuc-D-Luc have similar signal detection sensitivities (2.5 × 10³ cells). FIGs. 3H to 3M show the signal detection sensitivities of pTEAD1-mHRP-ABTS, pTEAD1-mHRP-TMB, pTEAD1-FLuc-D-Luc, pARE-mHRP-ABTS, pARE-mHRP-TMB, and pARE-FLuc-D-Luc, respectively. In the experiments of FIGs. 3H to 3M, 2500 ng of plasmids are serially diluted two-fold and then pTEAD1 or pARE containing mHRP or FLuc is transfected into the cells. In a "diluted total amount of plasmids sensitivity test assay," sensitivity is defined as the "minimum" total amount of plasmids required to produce a "discernible" signal. A comparison of pTEAD1-mHRP-ABTS (293T-TEAD1-mHRP+ABTS) (FIG. 3H), pTEAD1-mHRP-TMB (293T-TEAD1-mHRP+TMB) (FIG. 3I), and pTEAD1-FLuc-D-Luc (293T-TEAD1-FLuc+D-Luc) (FIG. 3J) reveal that pTEAD1-mHRP-TMB and pTEAD1-FLuc-D-Luc have similar signal detection sensitivities (total amount of 156.25 ng of plasmid). A comparison of pARE-mHRP-ABTS (293T-ARE-mHRP+ABTS) (FIG. 3K), pARE-mHRP-TMB (293T-ARE-mHRP+TMB) (FIG. 3L), and pARE-FLuc-D-Luc (293T-ARE-FLuc+D-Luc) (FIG. 3M) reveal that pARE-mHRP-TMB and pARE-FLuc-D-Luc have similar signal detection sensitivities (total amount of plasmids 78.13 ng). pTEAD1 group: number is 3, pARE group: number is 3, three independent replicate experiments are performed. All error bars represent standard deviations (SD). All x-axes are converted to a log₂ scale. Dynamic range (D.R.) is the ratio of the maximum signal to three times of the standard deviation.

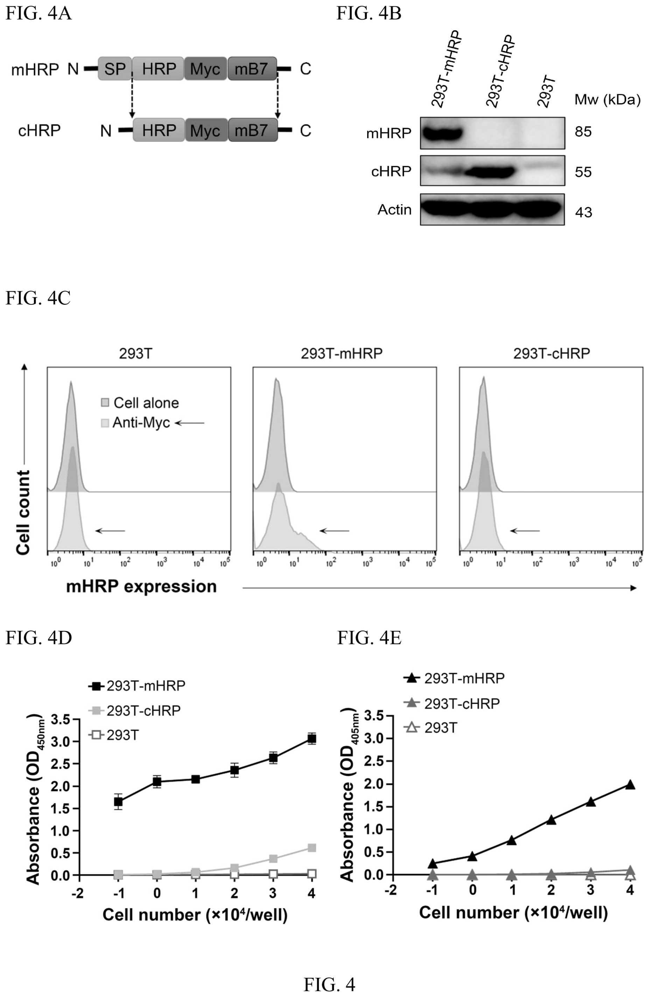

FIG. 4 shows that "membrane expression of HRP" is crucial in the mHRP reporter gene system. FIG. 4A is schematic diagrams of the mHRP and cHRP protein sequences. cHRP is produced by removing the signal peptide (SP) from mHRP. FIG. 4B shows that, after removing SP from mHRP, the molecular weight of mHRP changes from 85 kDa to 55 kDa of cHRP. Western blot analysis: Lane 1: 293T-mHRP; Lane 2: 293T-cHRP; Lane 3: 293T cells as a negative control (NC). Actin is used as a control group for total number of cells. FIG. 4C shows membrane expression of mHRP on 293T, 293T-mHRP, or 293T-cHRP cells. Analysis is done by using a flow cytometer, 293T cell (dark gray), 293T-mHRP cell, or 293T-cHRP cell alone is used as the control group for unstained antibodies, and anti-Myc antibodies and corresponding secondary antibodies (light gray, arrows indicate the groups). FIGs. 4D and 4E show the results of the reaction of 1.6×10⁵ of serially diluted (2-fold) 293T-mHRP or 293T-cHRP cells and substrates. TMB (FIG. 4D) or ABTS (FIG. 4E) of the 293T cells (hollow squares or hollow triangles), 293T-mHRP (dark solid squares or solid triangles), and 293T-cHRP (light solid squares or solid triangles) are detected at OD₄₅₀ₙₘ or OD₄₀₅ₙₘ, respectively. All x-axes are converted to a log₂ scale. Number is 3. Three independent replicate experiments are performed.

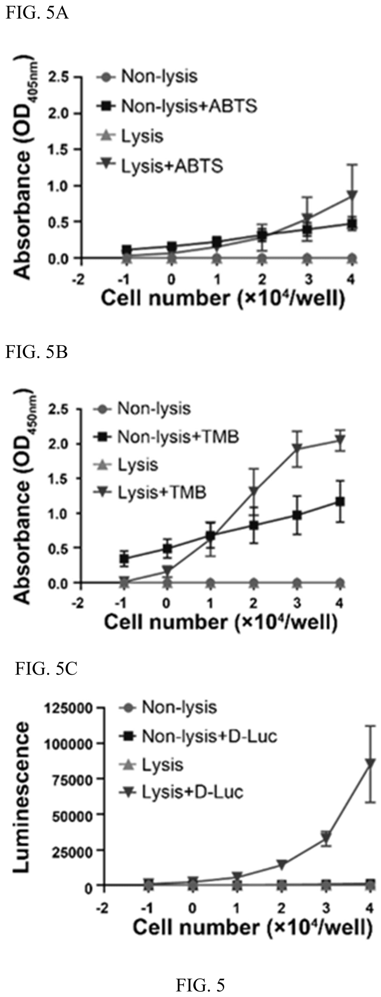

FIG. 5 shows that the "cell lysis step" is not essential for mHRP chromogenic assays, but is essential for firefly luciferase assays. 1.6×10⁵ of lysed or unlysed 293T cells for expressing mHRP or FLuc are serially diluted (2-fold dilution) and the OD₄₀₅ₙₘ, OD₄₅₀ₙₘ, or fluorescence is measured. FIG. 5A shows the pLNCX-CMV-ABTS group, where circles represent unlysed, squares represent unlysed +ABTS, equilateral triangles represent lysed, and inverted triangles represent lysed +ABTS. FIG. 5B shows the pLNCX-CMV-TMB group, where circles represent unlysed, squares represent unlysed +TMB, equilateral triangles represent lysed, and inverted triangles represent lysed +TMB. When the cells are unlysed, after reacting the mHRP cells with the substrate, a certain level of discernible signals can be generated; when the cells are lysed, after reacting the mHRP cells with the substrate, signals can still be generated, indicating that the "cell lysis step" is not essential for mHRP chromogenic assays. FIG. 5C shows the pLKO-CMV-D-Luc group. Circles represent unlysed, squares represent unlysed +D-Luc, equilateral triangles represent lysed, and inverted triangles represent lysed +D-Luc. When the cells are unlysed, after the FLuc cells react with D-Luc, almost no fluorescent signal is generated. When the cells are lysed, after the FLuc cells react with D-Luc, fluorescent signals can be generated, indicating that the "cell lysis step" is crucial to the firefly luciferase assay. Number is 4. 3 independent replicate experiments are performed. All error bars represent SD. All x-axes are converted to a log₂ scale.

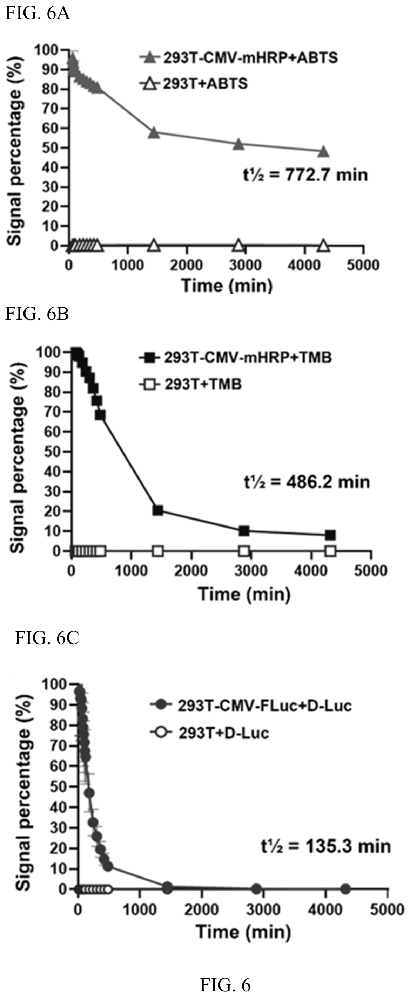

FIG. 6 shows that the signals from the mHRP chromogenic assay is more stable and lasting longer than the fluorescent signals of the FLuc assay. FIG. 6A shows the optical signal detection of 293T-CMV-mHRP+ABTS (solid triangles). FIG. 6B shows the optical signal detection of 293T-CMV-mHRP+TMB (solid squares). FIG. 6C shows the optical signal detection of 293T-CMV-FLuc+D-Luc (solid circles). Hollow triangles, hollow squares, and hollow circles represent 293T cells and the corresponding substrates. Measurements were taken every 10 minutes from 0 to 120 minutes, every 60 minutes from 120 to 480 minutes, and every 1440 minutes from 1440 to 4320 minutes. The t_{1/2} for 293T-CMV-mHRP+ABTS is 772.7 minutes, the t_{1/2} for 293T-CMV-mHRP+TMB is 486.2 minutes, and the t_{1/2} for 293T-CMV-FLuc+D-Luc is 135.3 minutes. Therefore, the signal from the mHRP chromogenic assay is more stable and lasting longer than the fluorescent signals from the FLuc assay. The t_{1/2} is calculated by using GraphPad nonlinear analysis - a plateau followed by one-phase decay. Number is 3. Three independent replicate experiments are performed.

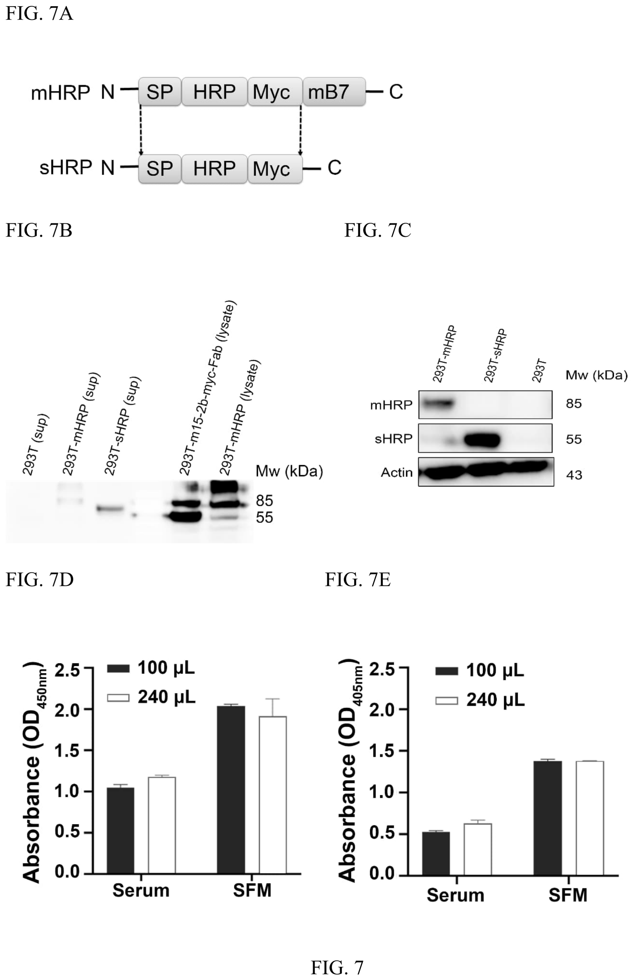

FIG. 7 shows that secreted horseradish peroxidase (sHRP) has the correct molecular weight of 55-72 kDa and enzyme activity. pLNCX-CMV-sHRP-myc is transiently transfected into 293T cells (the supernatant (sup) of the present invention is collected approximately 48 hours after transient transfection by removing the sup from the "6-well plate used for transient transfection," and increasing the centrifugation speed and time to minimize any interference from the cells). FIG. 7A shows a schematic diagram of the mHRP and sHRP protein sequences. sHRP is generated by removing mB7 from mHRP. FIG. 7B shows western blot analysis: Lane 1: 293T (sup); Lane 2: 293T-mHRP (sup); Lane 3: 293T-sHRP (sup); Lane 5: 293T-m15-2b-myc-Fab (lysate); Lane 6: 293T-mHRP (lysate). 293T (sup) serves as a negative control (NC). 293T-sHRP (sup) has the correct molecular weight of 55-72 kDa (the original molecular weight of sHRP is calculated to be 38 kDa; but glycosylation can increase the protein weight, which can result in a 55-72 kDa band). The 293T-mHRP (lysate) group serves as a positive control (PC) to confirm successful transient transfection. The 293T-m15-2b-myc-Fab (lysate) group shows expression and has the correct molecular weight of 67 kDa, serving as a positive control (PC) for stained antibodies. FIG. 7C shows western blot analysis (all 3 groups are analyzed by using lysates): Lane 1: 293T-mHRP; Lane 2: 293T-sHRP; Lane 3: 293T. The 293T-mHRP group serves as a positive control (PC) to confirm successful transient transfection. 293T-sHRP has the correct molecular weight of 55-72 kDa (the original molecular weight of sHRP is calculated to be 38 kDa; but glycosylation can increase the protein weight, which can result in a 55-72 kDa band). 293T serves as a negative control (NC). Actin serves as a control group for total number of cells. FIGs. 7D and 7E show that approximately 48 hours after transient transfection, 100 µL of sup was transferred to a 96-well plate and 100 µL of TMB or ABTS is added for chromogenesis, followed by detection of the signals by using an ELISA analyzer (for the 240 µL sup group, 240 µL of substrate is added to a 1.5 mL eppendorf tube for reaction, then transferred to a 96-well plate for signal detection). TMB (FIG. 7D) or ABTS (FIG. 7E) is detected at OD₄₅₀ₙₘ or OD₄₀₅ₙₘ, respectively, which show chromogenic reactions, indicating enzyme activities. SFM: serum-free media (DMEM + 1% P/S).

### EXAMPLES

The present invention can be implemented in many different forms and should not be construed as being limited to the embodiments set forth herein. Described embodiments are not intended to limit the scope of the present invention, as the scope described in the claims.

### Materials and methods

### Plasmid construction

The mHRP gene sequence was obtained by full gene synthesis and then subcloned into a pLNCX vector containing the myc-mB7 sequence. PCR was used to add 5' NcoI and 3' FseI restriction enzyme cutting sites to mHRP-myc-mB7, and then subcloned into the TEAD1 vector to create pTEAD1-mHRP-myc-mB7. 5' HindIII and 3' EcoRI restriction enzyme cutting sites were added to mHRP-myc-mB7 by PCR, and subcloned into an ARE vector to create pGL4.37[mHRP-myc-mB7/ARE/Hygro]. The signal peptide of pLNCX-Optimized mHRP-myc-mB7 was removed by PCR, and the signal peptide-removed mHRP-myc-mB7 was subcloned into a pLNCX vector by using the 5' HindIII and 3' SalI restriction enzyme cutting sites to create pLNCX-Optimized mHRP-myc-mB7 (without signal peptide).

### Cell culture

HEK 293T cells were cultured in DMEM (Sigma-Aldrich, Burlington, USA) + 10% BCS (Hyclone, Washington, USA) + 1% Penicillin/Streptomycin (Lonza, Basel, Switzerland) as growth medium. After being transfected for 24 hours, HEK 293T-[mHRP-myc-mB7/ARE/Hygro] cells and HEK 293T-[luc2P/ARE/Hygro] cells were added with 10 µM of tBHQ (Sigma-Aldrich, Burlington, USA), and then cultured at a final concentration of 0.1% v/v DMSO (Sigma-Aldrich, Burlington, USA). All cells were cultured in a 37°C and 5% CO₂ incubator.

### Transient transfection

293T cells (1 × 10⁶) were resuspended in 1.5 mL of growth medium and seeded into each well of a 6-well cell culture plate (Greiner Bio-One, Kremsmünster, Austria). After 16-24 hours, a total of 2.5 µg of plasmids (mHRP (or luciferase) plasmids: Renilla plasmids (as an internal control group) at a ratio of 40:1) was added to 125 µL of Opti-MEM (Gibco, Waltham, USA), and then mixed with "12 µL of Lipofectamine 2000 (Invitrogen, Waltham, USA) which was added to 125 µL of Opti-MEM" to obtain a transfected DNA-positively charged lipid microsome (lipofectamine) mixture. The culture medium was removed from the cultured cells and replaced it with 1 mL of fresh growth medium. A DNA-positively charged lipid microsome mixture was evenly added to each designated well and placed on a rotator at 40 rpm for 10 minutes. The transfected 6-well plate was incubated at 37°C and 5% CO₂ for 4 hours, replaced with 1.5 mL of fresh growth medium until assay time. Flow cytometry, western blot, ELISA, and dual-luciferase assay were performed 48 hours after transient transfection.

Comparison of signal detection sensitivity of mHRP and FLuc systems by diluting total number of cells

16-24 hours after transient transfection, TEAD1- or ARE-mHRP cells (luciferase cells) were serially diluted in 15 mL centrifuge tubes (Falcon, Corning, USA) from 1.6 × 10⁵ cells to 5 × 10³ cells (6 dilution points) or 2.5 × 10³ cells (7 dilution points, the cells were resuspended in a growth medium containing 10 µM of tBHQ, a final concentration of 0.1% v/v DMSO), 2-fold dilution. Each aliquot of cells was resuspended in 150 µL of culture medium and seeded into each well of a 96-well or white microplate (Greiner Bio-One, Kremsmünster, Austria) containing 50 µg/mL of poly-L-lysine (Sigma-Aldrich, Burlington, USA).

Comparison of signal detection sensitivities of mHRP and FLuc systems by diluting total amount of plasmids

Before transient transfection, plasmids were serially diluted with ultrapure water in 2-fold in a 0.5 mL centrifuge tube, from 2500 ng to 156.25 ng (5 dilution points, (TEAD1-mHRP (or luciferase) plasmid: Renilla plasmid = 40:1)) or from 2500 ng to 78.13 ng (6 dilution points, ARE-mHRP (or luciferase) plasmid: Renilla plasmid = 40:1), and then transfected into each well containing 1 × 10⁶ 293T cells. 16-24 hours after transient transfection, the cells transfected with mHRP or luciferase were seeded into a 96-well plate containing 50 µg/mL of poly-L-lysine (8 × 10⁴ cells per well, resuspended in 150 µL of culture medium). The signals were detected by using a Thermo Scientific^{™} Multiskan^{™} GO Microplate Spectrophotometer (Thermo Scientific, Waltham, USA) and a PerkinElmer Victor X2 Multilabel Microplate Reader (Perkin Elmer, Waltham, USA) to compare the sensitivities of the mHRP reporter gene and the luciferase reporter gene after transient transfection.

### mHRP reporter gene chromogenic assay

48 hours after transient transfection, the culture medium was removed from the cultured cells and washed with 200 µL of serum-free medium. ABTS (1 mg/mL, 150 µL, Sigma-Aldrich, Burlington, USA) and 0.01% hydrogen peroxide (Sigma-Aldrich, Burlington, USA) or TMB (0.1 mg/mL, 100 µL, Promega, Madison, USA) were added to each well, and shaken on a rotator at 50 rpm (room temperature). After TMB reacted for 45 minutes, the reaction was terminated by adding 100 µL of 1 M HCl (Sigma-Aldrich, Burlington, USA), and reacted again at room temperature for 15 minutes. The ABTS group reacted for 1 hour, no termination solution was added. The results were read by using a Multiskan^{™} GO microplate spectrophotometer, the absorbance of ABTS group was detected at 405 nm, and the absorbance of the TMB group was detected at 450 nm, 650 nm (background). The supernatant (100 µL) was then transferred to a new 96-well plate for signal detection without interference of the cells.

### Dual-luciferase assay

48 hours after transient transfection, 75 µL of supernatant was sucked out from the cultured cells. The experiments were performed using a dual-luciferase assay kit (Promega, Madison, TN, USA) according to the manufacturer's instructions. Reagent 1 (75 µL), containing cell lysis buffer solution and firefly luciferase substrate, or reagent 2 (75 µL), containing a firefly fluorescence-termination reagent and Renilla luciferase substrate, was added to each well and allowed to react at room temperature for 10 minutes. Firefly or Renilla fluorescence was detected by using a PerkinElmer Victor X2 Multilabel Microplate Reader (Perkin Elmer, Waltham, USA) (10 seconds for each well).

### Experiments for confirming whether mHRP and FLuc systems could generate signals by lysing cell or not

48 hours after transient transfection, the culture medium was sucked out from the cultured cells and the cells were washed with 200 µL of SFM. Glo Lysis Buffer (100 µL, Promega, Madison, USA) was added to each well of the lysed cell group, and PBS (100 µL) was added to each well of the unlysed cell group. The culture plate was shaken at 50 rpm for 5 minutes (room temperature). ABTS (150 µL) or PBS was added to the mHRP group, and shaken at 50 rpm for 1 hour at room temperature. The total volumes of the lysed cell and unlysed cell groups were the same, 250 µL. TMB (100 µL) or PBS was added to the mHRP group and shaken at 50 rpm at room temperature. After TMB reacted for 45 minutes, the reaction was terminated by adding 1 M of HCl (100 µL) or PBS was added, allowed to react again for 15 minutes at room temperature. The total volumes of the lysed cell and unlysed cell groups were the same, 300 µL. The results were read by using a Multiskan^{™} GO microplate spectrophotometer, the absorbance of the ABTS group was measured at 405 nm, and the absorbance of the TMB group was measured at 450 nm, 650 nm (background). The supernatant (100 µL) was transferred to a new 96-well plate for signal detection to protect the signals from being interfered by the cells. After being reacted on a rotator for 5 minutes (lysed cells or unlysed cells), the supernatant (75 µL) of the FLuc group was transferred to a new 96-well white plate, and 75 µL of Reagent 1 containing lysis buffer and firefly luciferase substrate (Promega, Madison, USA), or 75 µL of PBS was added to corresponding groups. Reagent 1 or PBS was added and reacted at room temperature for 10 minutes. Firefly fluorescence was detected by using a PerkinElmer Victor X2 Multilabel Microplate Reader, 10 seconds for each well. The total volumes of the lysed cell and unlysed cell groups were the same, 150 µL.

### Signal stability experiment

293T cells (1×10⁶ cells) were resuspended in 1.5 mL of growth medium and seeded in a 6-well cell culture plate. pLNCX-Optimized mHRP-myc-mB7 (or pLKO-AS2-luciferase-IRES-eGFP-zeocin) and pcDNA3.1-Renilla luciferase were transfected into to the 293T cells. The total amount of plasmids was 2.5 µg (CMV-mHRP (or CMV-luciferase) plasmids: Renilla plasmids = 40:1). After 16-24 hours, 1×10⁴ mHRP- or FLuc-expressing cells were seeded into 96-well plates or white plates (n = 3). The procedures of the reporter gene chromogenic assay and dual-luciferase assay were similar to those described above. For the TMB group, 1 M HCl (termination solution) was omitted, and the absorbance was detected at OD 370 nm. The FLuc group did not contain Reagent 2. The detection time was from 0 minute to 72 hours. During the first 2 hours, the signals were detected every 10 minutes. After 2 hours, the signals were detected every 1 hour. After 24 hours, the signals were detected every 24 hours. The Renilla fluorescence in two groups was measured for data correction.

### Flow cytometry

48 hours after transient transfection, cells were harvested and counted, and centrifuged at 300 g for 3 minutes (4°C). The cells were washed with 3 mL of cold PBS + 0.05% BSA (Sigma-Aldrich, Burlington, USA) in flow tubes (Falcon, Corning, USA). The cells (5 × 10⁵ cells) were harvested and stained with 200 µL, 1:200 (1.25 × 10⁻³ mg/mL) purified anti-c-myc antibodies (BioLegend, San Diego, USA) (resuspended in PBS + 0.05% BSA) and reacted for 45 minutes. The cells were then washed with 3 mL of cold PBS + 0.05% BSA and centrifuged at 500 g for 5 minutes (4°C, repeated twice). The cells were then stained with 200 µL of 1:200 (3.75 × 10⁻³ mg/mL) fluorescein (FITC) affiniPure goat anti-mouse IgG (Fcy specific fragment) (Jackson ImmunoResearch, Philadelphia, USA) (resuspended in PBS + 0.05% BSA), and reacted for 45 minutes (protected from light). The cells were washed with 3 mL of cold PBS + 0.05% BSA and centrifuged at 500 g for 5 minutes (4°C, repeated twice). The cells were resuspended in 300 µL of 1:100 propidium iodide (PI) (10 µg/mL, Sigma-Aldrich, Burlington, USA) (resuspended in PBS + 0.05% BSA), and passed through a cell strainer into a flow microtube. Green fluorescence was detected by using a flow cytometer (Guava easyCyte, Millipore, Burlington, USA), and analyzed by using the Guavasoft software.

### Western blot

The cells (1 × 10⁶ cells) were lysed in 50 µL of 6x Laemmli buffer containing 10% β-mercaptoethanol. The samples were boiled for 5 minutes and placed on ice for 5 minutes before analysis. Proteins were separated with 10% SDS-PAGE and transferred to a PVDF membrane (0.45 µm, Merck Millipore, Burlington, USA). The PVDF membrane was blocked with 5% skim milk for 1 hour, stained with 1:1000 (2.5 × 10⁻⁴ mg/mL) purified mouse anti-c-myc antibodies (BioLegend, San Diego, USA) (resuspended in skim milk). The PVDF membrane containing the sample was washed with PBST for 5 minutes (repeated three times), and then washed again with PBS for 5 minutes. Stained with 1:1000 (4 × 10⁻⁴ mg/mL) peroxidase-affiniPure goat anti-mouse IgG (Fc gamma specific fragment) (Jackson ImmunoResearch, Philadelphia, USA) (resuspended in skim milk). Washed with PBST for 5 minutes (repeated three times), and then washed with PBS for 5 minutes. The PBS was removed, and 1 mL of horseradish peroxidase (HRP) substrate peroxide solution and 1 mL of HRP substrate luminol reagent (Merck Millipore, Burlington, USA) were added. Chemiluminescent signals were detected by using a chemiluminescent Western Blot Imaging System (Azure Biosystems, Dublin, USA).

### sHRP supernatant (sup) enzyme activity test

2.5 µg of pLNCX-Optimized-sHRP-myc was transiently transfected into the 293T cells in a 6-well plate, 4 hours later, replaced with 1.5 mL of DMEM + 1% P/S (SFM) or DMEM + 10% BCS + 1% P/S. 48 hours after transient transfection, the supernatant was removed from the 6-well plate and centrifuged at 500 g for 5 minutes to minimize any interference from the cells. 100 µL of sHRP sup was transferred to a 96-well plate, 100 µL of ABTS was added and reacted for 1 hour or 100 µL of TMB was added and reacted for 45 minutes, 100 µL of 1M HCl was added to terminate the reaction, reacted again at room temperature for 15 minutes, shaken at 50 rpm (room temperature). The 240 µL sHRP sup group was reacted in a 1.5 mL centrifuge tube, 240 µL of ABTS was added to react for 1 hour or 240 µL of TMB was added to react for 45 minutes, 240 µL of 1M HCl was added to terminate the reaction, and reacted again at room temperature for 15 minutes, shaken at 50 rpm (room temperature). After 240 µL of the sHRP sup group was mixed thoroughly, 300 µL of the supernatant (from both groups) was transferred to a 96-well plate. The results were read by using a Multiskan^{™}GO microplate spectrophotometer, the absorbance of the ABTS group was detected at 405 nm, and the absorbance of the TMB group was detected at 450 nm, 650 nm (background). 100 µL of the supernatant was transferred to a new 96-well plate and the signals were detected again.

### Statistical analysis

GraphPad Prism 8.0.1 (GraphPad Software, San Diego, U.S.) was used to conduct statistical analysis of the experimental result of ELISA and dual-luciferase (or luciferase). FlowJo 10.8.1 was used to carry out the statistical analysis of the flow cytometry.

### Experimental results

FIG. 2A is a schematic diagram of the protein sequence of mHRP. The mHRP sequence was inserted into the pLNCX-CMV vector: starting from the N-terminus, the Igκ signal peptide, mHRP, Myc (tag extracellular domain), and mB7 (transmembrane and intracellular domain). FIG. 2B shows that 293T-mHRP was generated for transient expression of mHRP. Protein expression of mHRP was analyzed by western blot, Lane 1: 293T-mHRP; Lane 2: 293T cells served as a negative control (NC). Actin served as a control group for total number of cells. FIG. 2C shows membrane expression of mHRP detected by the Myc tag and corresponding secondary antibody, followed by a flow cytometry analysis. 293T-mHRP or 293T cells alone (dark gray) served as a control group for unstained antibodies, the anti-Myc antibody and corresponding secondary antibody were stained (light gray, arrows indicate the groups). FIGs. 2D and 2E show functional analysis of 293T-mHRP. 1.6×10⁵ 293T-mHRP cells were serially diluted (2-fold dilution), the 293T cells (hollow triangles or hollow squares) and 293T-mHRP (solid triangles or solid squares) were detected for ABTS (FIG. 2D) or TMB (FIG. 2E) at OD₄₀₅ₙₘ or OD₄₅₀ₙₘ, respectively. These results proved that the mHRP protein could be produced in the cells.

FIG. 3A shows a schematic diagram of the protein sequences of pTEAD1-mHRP, pTEAD1-FLuc, pARE-mHRP, and pARE-FLuc. The mHRP sequence (from SP to mB7) from pLNCX-CMV-mHRP was inserted into the pTEAD1 vector and pARE vector. FIGs. 3B to 3G show 2-fold dilutions (6 or 7 dilution points) of 1.6 × 10⁵ pTEAD1 (or pARE) cells containing mHRP or FLuc. For functional analysis of mHRP and FLuc, ABTS or TMB were detected at OD₄₀₅ₙₘ or OD₄₅₀ₙₘ, respectively, and D-Luc (D-luciferin) was detected at a preset fluorescence wavelength of 562 nm. Optical signals were detected by using an ELISA analyzer or a fluorimeter. The signals produced by reacting mHRP with TMB was higher than that produced with ABTS, indicating that TMB was more sensitive than ABTS. In the "diluted total number of cells sensitivity test assay," sensitivity was defined as the minimum total number of cells required to generate a discernible signal. A comparison of pTEAD1-mHRP-ABTS (293T-TEAD1-mHRP+ABTS) (FIG. 3B), pTEAD1-mHRP-TMB (293T-TEAD1-mHRP+TMB) (FIG. 3C), and pTEAD1-FLuc-D-Luc (293T-TEAD1-FLuc+D-Luc) (FIG. 3D) revealed that pTEAD1-mHRP-TMB and pTEAD1-FLuc-D-Luc had similar signal detection sensitivity (5×10³ cells). A comparison of pARE-mHRP-ABTS (293T-ARE-mHRP+ABTS) (FIG. 3E), pARE-mHRP-TMB (293T-ARE-mHRP+TMB) (FIG. 3F), and pARE-FLuc-D-Luc (293T-ARE-FLuc+D-Luc) (FIG. 3G) revealed that pARE-mHRP-TMB and pARE-FLuc-D-Luc had similar signal detection sensitivity (2.5×10³ cells). FIGs. 3H to 3M show that 2500 ng of plasmids were serially diluted two-fold and then pTEAD1 or pARE containing mHRP or FLuc was transfected into the cells. In the "diluted total amount of plasmid sensitivity test assay," sensitivity was defined as the minimum total amount of plasmids required to generate a discernible signal. A comparison of pTEAD1-mHRP-ABTS (293T-TEAD1-mHRP+ABTS) (FIG. 3H), pTEAD1-mHRP-TMB (293T-TEAD1-mHRP+TMB) (FIG. 3I), and pTEAD1-FLuc-D-Luc (293T-TEAD1-FLuc+D-Luc) (FIG. 3J) revealed that pTEAD1-mHRP-TMB and pTEAD1-FLuc-D-Luc had similar signal detection sensitivity (total amount: 156.25 ng of plasmid). A comparison of pARE-mHRP-ABTS (293T-ARE-mHRP + ABTS) (FIG. 3K), pARE-mHRP-TMB (293T-ARE-mHRP + TMB) (FIG. 3L), and pARE-FLuc-D-Luc (293T-ARE-FLuc + D-Luc) (FIG. 3M) revealed that pARE-mHRP-TMB and pARE-FLuc-D-Luc had similar signal detection sensitivity (total amount: 78.13 ng of plasmid). These results proved that the mHRP expressed in cells possessed the desired function and exhibits comparable dynamic range and sensitivity to fluorescence.

FIG. 4A shows a schematic diagram of the protein sequences of mHRP and cHRP. cHRP was produced by removing the SP from mHRP. FIG. 4B shows that after removing the SP from mHRP, the molecular weight of mHRP changed from 85 kDa to 55 kDa of cHRP. Western blot analysis: Lane 1: 293T-mHRP; Lane 2: 293T-cHRP; Lane 3: 293T cells served as a negative control (NC). Actin served as a control group for total number of cells. FIG. 4C shows the membrane expression of mHRP in 293T, 293T-mHRP, or 293T-cHRP cells. Flow cytometric analysis was carried out by using 293T, 293T-mHRP, or 293T-cHRP cells alone as a control group for unstained antibodies (dark gray), and anti-Myc antibodies and the corresponding secondary antibody groups were stained (light gray, arrows indicate the groups). FIGs. 4D and 4E show that 1.6×10⁵ 293T-mHRP or 293T-cHRP cells were serially diluted (2-fold) and reacted with substrates. 293T cells (hollow squares or hollow triangles), 293T-mHRP (dark solid squares or solid triangles), and 293T-cHRP (light solid squares or solid triangles) were detected for TMB (FIG. 4D) or ABTS (FIG. 4E) at OD₄₅₀ₙₘ or OD₄₀₅ₙₘ, respectively. These results proved the importance of the extracellular localization of mHRP itself.

FIG. 5A shows the pLNCX-CMV-ABTS group, where circle represents unlysed, square represents unlysed +ABTS, equilateral triangle represents lysed, and inverted triangle represents lysed +ABTS. FIG. 5B shows the pLNCX-CMV-TMB group, circle represents unlysed, square represents unlysed +TMB, equilateral triangle represents lysed, and inverted triangle represents lysed +TMB. When the cells were unlysed, after the mHRP cells reacted with the substrate, a certain level of discernible signals could be generated; when the cells were lysed, after the mHRP cells reacted with the substrate, signals could still be generated, indicating that the "cell lysis step" was not essential to the mHRP chromogenic reaction. FIG. 5C shows the pLKO-CMV-D-Luc group, where circle represents unlysed, square represents unlysed + D-Luc, equilateral triangle represents lysed, and inverted triangle represents lysed + D-Luc. When the cells were unlysed, after the FLuc cells reacted with D-Luc, almost no fluorescent signal was generated; when the cell were lysed, after the FLuc cells reacted with D-Luc, fluorescent signals were generated, indicating that the "cell lysis step" was crucial to the firefly luciferase assay. These results compared the necessity and differences of the cell lysis step between mHRP and fluorescent systems.

FIG. 6A shows optical signal detection of 293T-CMV-mHRP + ABTS (solid triangles), FIG. 6B shows optical signal detection of 293T-CMV-mHRP + TMB (solid squares), and FIG. 6C shows optical signal detection of 293T-CMV-FLuc + D-Luc (solid circles). Hollow triangles, hollow squares, and hollow circles represent the 293T cells and the corresponding substrates. The detections were undertaken every 10 minutes from 0 to 120 minutes, every 60 minutes from 120 to 480 minutes, and every 1440 minutes from 1440 to 4320 minutes. The t_{1/2} for 293T-CMV-mHRP+ABTS was 772.7 minutes, the t_{1/2} for 293T-CMV-mHRP+TMB was 486.2 minutes, and the t_{1/2} for 293T-CMV-FLuc+D-Luc was 135.3 minutes. Therefore, the signals of the mHRP chromogenic reaction were more stable and lasting longer than the fluorescence signals of the FLuc assay. The results proved that the substrate stability of mHRP significantly exceeded the stability of the fluorescence system.

FIG. 7A shows a schematic diagram of the protein sequences of mHRP and sHRP. sHRP was generated by removing mB7 from mHRP. FIG. 7B shows western blot analysis: Lane 1: 293T (sup); Lane 2: 293T-mHRP (sup); Lane 3: 293T-sHRP (sup); Lane 5: 293T-m15-2b-myc-Fab (lysate); Lane 6: 293T-mHRP (lysate). 293T (sup) served as a negative control (NC). 293T-sHRP (sup) had the correct molecular weight of 55-72 kDa (the original molecular weight of sHRP was calculated to be 38 kDa; but glycosylation could increase the protein weight, which could result in a 55-72 kDa band). The 293T-mHRP (lysate) group served as a positive control (PC) to confirm successful transient transfection. The 293T-m15-2b-myc-Fab (lysate) group expressed and had the correct molecular weight of 67 kDa, serving as a positive control (PC) for stained antibodies. FIG. 7C shows western blot analysis (all three groups were analyzed using lysates): Lane 1: 293T-mHRP; Lane 2: 293T-sHRP; Lane 3: 293T. The 293T-mHRP group served as a positive control (PC) to confirm successful transient transfection. 293T-sHRP had the correct molecular weight of 55-72 kDa (the original molecular weight of sHRP was calculated to be 38 kDa; but glycosylation could increase the protein weight, which could result in a 55-72 kDa band). The 293T cells served as a negative control (NC). Actin served as a control group for total number of cells. FIGs. 7D and 7E show that approximately 48 hours after transient transfection, 100 µL of sup was transferred to a 96-well plate and 100 µL of TMB or ABTS was added for chromogenesis, and an ELISA analyzer was used for signal detection (for the 240 µL sup group, 240 µL of substrate was added to a 1.5 mL eppendorf tube for reaction, and then transferred to a 96-well plate for signal detection.) TMB (FIG. 7D) or ABTS (FIG. 7E) were detected at OD₄₅₀ₙₘ or OD₄₀₅ₙₘ, respectively, which showed chromogenic reactions, indicating enzyme activities. These results proved that sHRP had correct protein expression and enzyme activity.

## Claims

1. A cell having a reporter gene system of a dual-structure chromogenic enzyme, which comprises a vector containing a polynucleotide, wherein the polynucleotide comprises a first gene set and a second gene set, the first gene set comprises a first promoter, a nucleic acid sequence encoding a first signal peptide, a nucleic acid sequence encoding a first chromogenic enzyme, and a nucleic acid sequence encoding a transmembrane domain; and the second gene set comprises a second promoter, a nucleic acid sequence encoding a second signal peptide, and a nucleic acid sequence encoding a second chromogenic enzyme.

2. The cell of claim 1, wherein the cell comprises a mammalian cell.

3. The cell of claim 1, wherein the first promoter and the second promoter are inducible promoters or continuously expressed promoters.

4. The cell of claim 1, wherein the first chromogenic enzyme and the second chromogenic enzyme comprise horseradish peroxidase, alkaline phosphatase, glucose oxidase, β-lactamase, or β-galactosidase.

5. The cell of claim 1, wherein the transmembrane domain comprises mB7, CD63, CD81, CD9, CD82, CD44, CD47, CD55, LAMP2B, ICAM, integrin, ARRDC1 or annexin.

6. A method for screening a candidate to be tested by using a cell having a reporter gene system of a dual-structure chromogenic enzyme, which comprises:
(1) providing a cell having a reporter gene system of a dual-structure chromogenic enzyme, and placing the cell in a culture medium, wherein the cell comprises a vector containing a polynucleotide, and the polynucleotide comprises a first gene set and a second gene set, wherein the first gene set comprises a first promoter, a nucleic acid sequence encoding a first signal peptide, a nucleic acid sequence encoding a first chromogenic enzyme, and a nucleic acid sequence encoding a transmembrane domain; and the second gene set comprises a second promoter, a nucleic acid sequence encoding a second signal peptide, and a nucleic acid sequence encoding a second chromogenic enzyme;
(2) contacting at least one candidate to be tested with the cell, wherein the at least one candidate to be tested promotes expression of the first promoter and the second promoter in the polynucleotide, and encodes the following proteins: the first signal peptide, the first chromogenic enzyme, the transmembrane domain, the second signal peptide, and the second chromogenic enzyme, wherein the first signal peptide and the transmembrane domain cause the first chromogenic enzyme to be expressed on the cell membrane of the cell, and the second signal peptide causes the second chromogenic enzyme to be released from the cell into the culture medium;
(3) centrifuging the culture medium to separate a separated solution containing the cell expressing the first chromogenic enzyme on the cell membrane and a supernatant containing the second chromogenic enzyme;
(4) adding a first substrate to the separated solution to perform a first chromogenic reaction with the first chromogenic enzyme, and adding a second substrate to the supernatant to perform a second chromogenic reaction with the second chromogenic enzyme; and
(5) detecting signals of the first chromogenic reaction and the second chromogenic reaction, and selecting the signals of at least one chromogenic reaction to analyze characteristics of the at least one candidate to be tested.

7. The method of claim 6, wherein the cell comprises a mammalian cell.

8. The method of claim 6, wherein the first chromogenic enzyme and the second chromogenic enzyme comprise horseradish peroxidase, alkaline phosphatase, glucose oxidase, β-lactamase, or β-galactosidase.

9. The method of claim 6, wherein the at least one candidate to be tested comprises a compound or a protein.

10. The method of claim 6, wherein the first substrate and the second substrate comprise 2,2'-azino-bis(3-ethylbenzothiazoline-6-sulfonic acid), 3,3',5,5'-tetramethyl-[1,1'-biphenyl]-4,4'-diamine, 3,3'-diaminobenzidine tetrahydrochloride, 4-chloro-1-naphthol, 3-amino-9-ethyl carbazole, enhanced chemiluminescence solution, or luminol.

11. The method of claim 6, wherein when the at least one candidate to be tested is one single candidate to be tested, the step (5) comprises selecting signals from one of the chromogenic reactions for analyzing cell transfection efficiency of the vector; and signals from the other chromogenic reaction for analyzing characteristics of the at least one candidate to be tested.

12. The method of claim 6, wherein when the at least one candidate to be tested comprises a first candidate to be tested and a second candidate to be tested, the step (5) comprises selecting signals from one of the chromogenic reactions for analyzing characteristics of the first candidate to be tested, and signals from the other chromogenic reaction for analyzing the characteristics of the second candidate to be tested.

13. The method of claim 6, wherein the step (5) further comprises determining whether the at least one candidate to be tested is a promoting molecule or an inhibitory molecule based on a characteristic analysis of the at least one candidate to be tested, wherein the determination method is that when the signals of the chromogenic reaction of the at least one candidate to be tested are higher than signals of a chromogenic reaction of a control group, the at least one candidate to be tested is a promoting molecule; otherwise, when the signals of the chromogenic reaction of the at least one candidate to be tested are lower than the signals of the chromogenic reaction of the control group, the at least one candidate to be tested is an inhibitory molecule.
